Europäisches Patentamt

European Patent Office

Office européen des brevets

(11) Veröffentlichungsnummer: **0 137 978 B1**

(12) **EUROPÄISCHE PATENTSCHRIFT**

(45) Veröffentlichungstag der Patentschrift: **08.04.92**

(21) Anmeldenummer: **84109987.2**

(22) Anmeldetag: **22.08.84**

(51) Int. Cl.5: **A61K 47/00, A61K 31/21**

(54) **Zubereitung von Nitroestern für die Koronartherapie.**

(30) Priorität: **17.09.83 DE 3333639**

(43) Veröffentlichungstag der Anmeldung:
**24.04.85 Patentblatt 85/17**

(45) Bekanntmachung des Hinweises auf die
Patenterteilung:
**08.04.92 Patentblatt 92/15**

(84) Benannte Vertragsstaaten:
**AT BE CH DE FR GB IT LI LU NL SE**

(56) Entgegenhaltungen:
**EP-A- 0 108 218**
**FR-A- 1 555 348**
**GB-A- 1 315 197**
**US-A- 3 096 242**

(73) Patentinhaber: **Dynamit Nobel Aktiengesellschaft
Postfach 12 61
W-5210 Troisdorf(DE)**

(72) Erfinder: **Heinzelmann, Walter, Dr.
Neschener Str. 62
W-5068 Odenthal(DE)**

(74) Vertreter: **von Kreisler, Alek, Dipl.-Chem. et al
Patentanwälte Von Kreisler-Selting-Werner,
Deichmannhaus am Hauptbahnhof
W-5000 Köln 1(DE)**

**Beschreibung**

Nitroester, d.h. Nitrate von mehrwertigen aliphatischen oder zyklischen Alkoholen, dienen in der Koronartherapie, d.h. bei der Behandlung u.a. der Angina pectoris zur Langzeittherapie, der Anfallsprophylaxe wie auch der Anfallstherapie.

Üblich sind orale und besonders transdermale Applikationsformen, wobei letztere über die als Speicher- und Steuermembran wirkende Haut eine direkte und systemische Wirkung auf das Gefäßsystem sowie eine gleichmäßige Wirkstoffabgabe der Nitroester erlauben.

Während Spray-Zubereitungen überwiegend zur akuten Anfallstherapie gedacht sind, bewirken Salbenzubereitungen, z.B. auf der Basis von Ölen/Wasser-Emulsionen, und Gel-Zubereitungen durch die Art der Trägerbasis eine Retard-Wirkung und zusätzlich regulierende Freisetzung der NitroesterWirkstoffe.

Besonders thixotrope Gele mit hautpenetrierenden Ölen enthalten einerseits das Öl als Transportmittel des Wirkstoffs durch die Haut und bewirken andererseits durch das Thixotropierungsmittel und ggf. weiteren Trägerstoffen eine gleichmäßige Abgabe des Wirkstoffs aus dem Gel auf die oder in der Hautoberfläche.

Die vorliegende Erfindung betrifft Gel-Zubereitungen von koronarwirksamen Nitroestern.

Es ist bekannt, Nitroester der nachfolgend näher beschriebenen Art in Lösung von Ölen, beispielsweise fetten Ölen, mit beispielsweise disperser Kieselsäure zu Gelen von streichfähiger Konsistenz zu verarbeiten, deren Viskosität nur eine geringe Abhängigkeit von der Temperatur aufweist.

Nachteilig ist jedoch bei längerer Standzeit der Gele in Verpackungen wie Kapseln, Salbengefäßen oder Tuben das Austreten von flüssigen Abscheidungen des Öls, die bei der Anwendung stören und die gleichmäßige Dosierung der Wirkstoffe beeinträchtigen sowie aus der Verpackung austreten können.

Es bestand daher die Aufgabe, die Abscheidung von Ölen in wirkstoffhaltigen Gel-Zubereitungen zuverlässig zu verhindern, d.h. das sogenannte "Ausölen" zu vermeiden.

Gegenstand der Erfindung sind daher ohne Zusatz von Wasser hergestellte Gel-Zubereitungen bzw. gelhaltige Zubereitungen von Nitroestern sowie ein Verfahren zu deren Herstellung nach den Patentansprüchen. Erfindungsgemäß wird als feindisperses $SiO_2$ zur Herstellung der Gele ein hydrophobes $SiO_2$ neben dem üblichen hydrophilen $SiO_2$ verwendet.

Die Zubereitungen sind bevorzugt thixotrope Gele in transparenter Erscheinung bis ggf. durchscheinendem Aussehen mit Gehalte von gelösten Nitroestern. Bei Anwesenheit zusätzlicher fester Trägerstoffe für Nitroester ergehen sich nicht in allen Fällen transparente Gele. Bei Anwesenheit von festen Anteilen z.B. Trägerstoffen und/oder von Salbengrundlagen ergeben sich Zubereitungen mit Gehalten der Gele, in denen der Gehalt von hydrophobem $SiO_2$ das Ausölen der Ölbasis oder von flüssigen Nitroestern verhindert.

Je nach Mengenverhältnis der Ölbasis zu dem feindispersen $SiO_2$ besitzen die Zubereitungen eine stufenlos einstellbare variable Viskosität von ölig über streichfähig bis stichfest.

Demgemäß sind die Zubereitungen für verschiedene Applikationsformer geeignet, beispielsweise als Salbe bzw. Hautöl oder als ölartige bis pastenartige Füllung von Kaukapseln aus z.B. Weichgelatine, als streichfähige Gel-Zubereitung, als gelartig-plastische Füllung von Depot-Pflastern oder schließlich als ölige oder mehr oder minder steif-feste Füllung von Schluckkapseln aus z.B. Hart- oder Weichgelatine und dgl.

Wasser soll in den Zubereitungen nicht enthalten sein.

Der Gehalt von hydrophobem $SiO_2$ unterdrückt das Ausölen, so daß kein Ausölen während der gesetzlichen Haltbarkeitszeit auftritt. Es zeigt sich jedoch, daß der Gehalt von hydrophober Kieselsäure auch in der Lage ist, neben einer wesentlich verbesserten Lagerbarkeit auf lange Zeit auch eine Zersetzung des Nitroesters durch Zutritt von Feuchtigkeit zu vermindern.

Die erfindungsgemäßen wasserfreien Gel-Zubereitungen von Nitroestern, die kurz als Oleo-Gele bezeichnet werden können, enthalten im allgemeinen ein Verhältnis 0,5 bis 25,0 Gew.-Teilen des Wirkstoffes zu 55 bis 90 Gew.-Teilen einer Ölbasis zu 5,0 bis 40 Gew.-Teilen feindisperses $SiO_2$, wobei das feindisperse $SiO_2$ zu 95 bis 60 Gew.-% aus hydrophilem $SiO_2$ und zu 5 bis 40 Gew.-% aus hydrophobem $SiO_2$ besteht; vorzugsweise sind 20 bis 40 Gew.-% des $SiO_2$ hydrophobes $SiO_2$. Die Zubereitungen enthalten ggf. noch kleine oder großere Mengen Hilfsstoffe.

Bei Vorliegen der bevorzugten Lösungen von Nitroestern liegt der Gehalt dieser Nitroester in der Zubereitung im Falle leichter löslicher Nitroester wie Glycerintrinitrat bei 0,5 bis 25,0, vorzugsweise 0,5 bis 10 Gew.-%, jedoch bei schwerer löslichen Nitroestern wie Isosorbid-mono (oder di)-nitrat bei 0,5 bis 10,0 Gew.-%. Sollen höhere Konzentrationen von schwerer löslichen Nitroestern hergestellt werden, können diese in der Ölbasis entweder in reiner Form suspendiert oder oder absorbiert an feste Trägerstoffe suspendiert werden.

Weitere Stoffe in kleinen ggf. auch größere Mengen können den Zubereitungen zugesetzt werden, beispielsweise inerte Trägerstoffe für flüssige oder feste Nitroester.

Zubereitungen aus Lösungen der Nitroester enthalten diese vorzugsweise in Mengen von 0,5 bis 5,0 Gew.-% der Zubereitung. Das Verhältnis hydrophiles $SiO_2$ zu hydrophobem $SiO_2$ soll bevorzugt 80 bis 60 zu 20 bis 40 Gew.-% betragen.

Feindisperses hydrophiles $SiO_2$ wird üblicherweise durch Flammpyrolyse von $SiCl_4$ gewonnen. Das hydrophile $SiO_2$ besteht nicht ausschließlich aus dem Oxid, sondern enthält zumindest an der Oberfläche teilweise auch Silanolgruppen. Die kugeligen Primarteilchen des durch Pyrolyse hergestellten $SiO_2$ liegen im allgemeinen im Bereich von 5 bis 30 nm und bilden teilweise Sekundär-Agglomerate, welche das thixotrope Gelgerüst aufbauen.

Das hydrophobe feindisperse $SiO_2$ wird aus dem hydrophilen feindispersen $SiO_2$ durch Behandlung mit einem Hydrophobierungsmittel hergestellt, wobei verschiedene Hydrophobierungsmittel in Frage kommen, jedoch bevorzugt Alkylchlorsilane verwendet werden. Von den Alkylchorsilanen sind die Dichlordialkylsilane mit Gehalten von besonders Methylgruppen oder ggf. Ethylgruppen bevorzugt, obgleich auch Monochlortrialkylsilane und Trichlormonoalkylsilane mit bevorzugt Methylgruppen oder ggf. Ethylgruppen in Frage kommen.

Besonders ist das hydrophobe $SIO_2$ das Produkt R 972[R] der Firma Degussa AG, Frankfurt/M., welches in "Die pharmazeutische Industrie" 27 (1965), Seiten 300/301 beschrieben wird.

Als Ölbasis können an sich übliche pharmazeutisch verwendbare Öle verwendet werden, welche zwischen - 10º und + 30º, zumindest jedoch zwischen 0 und 30 ºC keine Trübungen aufweisen, nicht ranzig werden und in wasserfreiem Zustand praktisch nicht bakteriell angreifbar sind. Soweit daher Fettsäurereste in diesen Ölen enthalten sind, soll es sich um gesättigte Fettsäurereste handeln.

Als Ölbasis kommen daher flüssige Medien der genannten Eigenschaften in Frage, beispielsweise gesättigte Triglyceride oder Partialglyceride, flüssige Diole wie Propandiol-1,2 bzw. flüssige Polyole, Paraffinöle, flüssige Veresterungsprodukte der 2-, 3- und 4-wertigen Alkohole mit gesättigten geradkettigen oder verzweigten Fettsäuren und ggf. weiteren Anteilen von Dicarbonsäuren sowie flüssige Ester der niederen Fettalkohole mit niederen oder mittelkettigen Fettsäuren, weitere entsprechende Substanzen und deren Mischungen.

Bevorzugt besteht die genannte Ölbais aus flüssigen Triglyceriden von Mischungen gesättigter, geradkettiger Fettsäuren der Kettenlänge $C_8$ bis $C_{12}$, die ggf. mit flüssigem Paraffinöl gemischt sein können. Geeignet sind weiter 1,2- und/oder 1,3-Propandiol oder Propandiol-Fettsäureester der Fettsäuren der Kettenlänge $C_8$ bis $C_{12}$ sowie Umsetzungsprodukte von Partialglyceriden gesättigter Fettsäuren der Kettenlänge $C_6$ bis $C_{12}$ mit gesättigten Dicarbonsäuren wie Bernsteinsäure, Malonsäure oder Glutarsäure.

Sehr bevorzugt besteht die Ölbasis aus Triglyceriden gesättigter Fettsäuren der Kettenlänge $C_8$ bis $C_{12}$ oder deren Mischungen mit flüssigem Paraffinöl, aus 1,2- und/oder 1,3-Propandiol oder aus Umsetzungsprodukten von Partialglyceriden, besonders Dyglyceriden der gesättigten Fettsäuren mit 8 bis 12 C-Atomen mit Bernsteinsäure.

Als koronarwirksame Nitroester kommen besonders Glycerintrinitrat, die als Isosorbiddinitrat bzw. Isosorbid-5-mononitrat bezeichneten Nitroester des 1.4; 3.6-Dianhydrosorbids, Pentaerythrit-tetranitrat sowie deren Mischungen in Frage.

Als ggf. anwesende feste Trägerstoffe für die Nitroester können beispielsweise organische Trägerstoffe wie Glukose, Lactose, Sorbit, Mannit, feste Glyceride, kristalline Zellulosen, Stärkepräparationen o. dgl. sowie auch anorganische Trägerstoffe wie Cellite, Dicalciumphosphat, Aluminiumoxid, Titandioxid in zusätzlichen Mengen bis zu 85 Gew-%, vorzugsweise bis 60 Gew-%, der Zubereitung anwesend sein. Es ist dann zu verstehen, daß das Mengenverhältnis der Ölbasis (b) zu $SiO_2$ (c) und das Mengenverhältnis $C_1$ zu $C_2$ dem des Anspruchs 1 entspricht. Die Menge des Nitroesters kann aber bis 25 Gew-% der Zubereitung einschließlich des festen Trägerstoffs betragen.

Es soll verstanden werden, daß in kleineren Mengen in der Zubereitung Hilfsstoffe, UV-Stabilisatoren, Antioxidantien, Konservierungsmittel, Hautpflegemittel, Duftstoffe, Farbstoffe, Netzmittel u. ä. enthalten sein können.

Weiterhin können in der Ölbais kleine Mengen von Estern weiterer Fettsäuren sowie weiterer Polyole, einwertige Alkohole oder deren Ester enthalten sein.

Der Gehalt von hydrophober feindisperser $SiO_2$ bewirkt überraschend nicht nur eine verbesserte Langzeitlagerung und unterdrückt nicht nur das Ausölen der Ölbasis, sondern trägt auch zur Stabilisierung der Zubereitungen bei und verhindert insbesondere den Zutritt von Wasserdampf und vermindert entscheidend die dadurch möglichen

Entmischungserscheinungen. Die bei der Herstellung gewählte Viskosität bleibt auf lange Zeit genau erhalten. Die Hydrolyse der Nitroester sowie bakteriell bedingte Zersetzungserscheinungen treten auch bei langer Lagerung unter z.B. tropischen Bedingungen nicht auf. Ein Zusatz von Bakteriziden zur Verhinderung des bakteriellen Zerfalls der Zubereitungen erwies sich bei praktisch wasserfreien Zubereitungen als nicht

EP 0 137 978 B1

erforderlich.

Die Herstellung der Gel-Zubereitungen erfolgt im allgemeinen durch Vermischung der Bestandteile in evakuierbaren Rührwerken bei Normaldruck oder ggf. im Vakuum entsprechend Patentanspruch 8.

Hierbei geht man bevorzugt von Lösungen der flüssigen oder festen Nitroester in der Ölbasis aus, welche phlegmatisierte und somit handhabungssichere Zubereitungen der sprengstoffgefährlichen Nitroester darstellen.

Auch die Suspensionen des Nitroesters oder Suspensionen des an einen Trägerstoff absorbierten Nitroesters in der Zubereitung sind phlegmatisiert und gefahrlos bei der Herstellung der Zubereitungen handhabbar.

Der Zusatz von hydrophilem feindispersen $SiO_2$ sowie von hydrophobem feindispersen $SiO_2$ erfolgt getrennt oder gemeinsam kontinuierlich oder in einer oder mehreren Portionen unter Einrühren in Mengen, welche die gewollte Viskosität bis zu gleichbleibenden Meßwerten ergeben.

Zur Entfernung von Gaseinschlüssen rührt man die Zubereitungen anschließend unter vermindertem Druck bis zur vollständigen Entgasung.

Ein weiteres bevorzugtes Gemisch gemäß der Erfindung enthält 3 Gew.-% eines Triglycerids der gesättigten Fettsäuren Capryl-/Caprinsäure und 2 Gew.-% Paraffinöl als Ölbasis, 3 Gew.-% hydrophiles feindisperses $SiO_2$ und 2 Gew.-% hydrophobes feindisperses $SiO_2$, 9 Gew.-% Glycerintrinitrat als Nitroester sowie 81 Gew.-% Lactose als Trägerstoff.

Eine noch weitere Zubereitung gemäß der Erfindung enthält 94,9 Gew.-% eines Triglycerids der gesättigten Fettsäuren Capryl-/Caprinsäure und 1,4 Gew.-% eines Triglyceridgemisches gesättigter Pflanzenfettsäuren als Ölbasis, 1,2 Gew.-% hydrophiles feindisperses $SiO_2$, 0,5 Gew.-% hydrophobes feindisperses $SiO_2$ sowie 2 Gew.-% Isosorbit-Dinitrat als Nitroester.

Beispiele 1 bis 3 und Vergleichsbeispiel A bis D

Zur Herstellung der Gel-Zubereitungen der genannten Beispiele und Vergleichsbeispiele wird in einem evakuierbaren Salbenrührwerk die Ölbasis vorgelegt und darin der Nitroester bei 20 °C bzw. bis 35 °C durch Rühren verteilt. Die angegebenen Mengen an feindisperser $SiO_2$ sowohl dem üblichen hydrophilen Art wie auch das hydrophobe $SiO_2$ wird durch kurzes Rühren in der Mischung verteilt.

Zur Entlüftung wird unter vermindertem Druck (10 - 30 Torr) bis zu 2 Stunden weiter gerührt bis die Mischung frei von Luftblasen ist.

Die Zubereitungen sind klar transparente Gele.

Im Lagertest weisen die Zubereitungen nach den erfindungsgemäßen Beispielen keine Abscheidung der Ölbasis auf, während die Zubereitung der Vergleichsbeispiele nach 6 Monaten Anteile der Ölbasis aus dem Gel abgeschieden haben.

Bei Lagerung, Transport oder nach Abpackung der Gele in verkaufsfertiger Form werden bei den erfindungsgemäßen Zubereitungen keinerlei Abscheidungen der Ölbasis beobachtet.

Im Gegensatz dazu können bei den Zubereitungen nach den Vergleichsbeispielen A bis D ölige Abscheidungen durch Entmischung, besonders bei längeren Standzeiten und erhöhter Temperatur auftreten.

Beispiele 4 und 5

Die Zubereitungen der Beispiele 4 und 5 von salbenartiger Konsistenz werden in einem heizbaren und evakuierbaren Salbenrührwerk hergestellt. Hierzu wird Miglyol 812 bzw. 829 vorgelegt und der Nitroester durch Rühren bei Raumtemperatur darin gelöst.

Die entstandene Mischung wird auf 45ºC erwärmt und die weiteren Bestandteile der Ölbasis unter Rühren gelöst. Darauf werden die beiden Typen der feindispersen Kieselsäuren nacheinander oder gleichzeitig bei 45ºC zugegeben und die Zubereitung unter Abkühlung wie in den vorangehenden Beispielen fertiggestellt.

Die Stabilität im Stabilitätstest und in der praktischen Handhabung gibt zu keinerlei Beanstandung durch Ausölen Anlaß.

Beispiel 6 bis 8

Die Zubereitungen der Beispiele 6 bis 8 werden entsprechend der Verfahrensweise hergestellt, die für die Beispiele 4 bis 5 angegeben ist.

Die Zubereitung nach Beispiel 6 von salbenartiger Konsistenz werden in einem heizbaren und

4

evakuierbaren Salbenrührwerk hergestellt. Hierzu wird Miglyol-812 bzw. -829 vorgelegt und durch Beheizen mit Warmwasser unter Rühren auf 45°C temperiert und zuerst der feste Nitroester Isorbid-dinitrat, danach die weiteren Bestandteile der Ölbasis unter Rühren bei 45°C darin gelöst. Darauf werden die beiden Typen der feindispersen Kiesel säure nacheinander oder gleichzeitig bei 45°C zugegeben und die Zubereitung unter Abkühlung wie in den vorangehenden Beispielen fertiggestellt.

Die Zubereitungen der Beispiele 7 und 8 erfolgen entsprechend den Angaben für die Beispiele 4 und 5, jedoch wird der Nitroester gemeinsam mit dem festen Trägerstoff am Schluß zugegeben. Dazu wird bei 20°C oder unter Kühlung in einem separaten Arbeitsgang in einem Rührwerk gepulverte Lactose vorgelegt und in Portionen Glyerintrinitrat zugegeben und gleichmäßig in der Lactose verteilt, wobei eine Absorbierung des Glycerintrinitrats an die Lactose auftritt (Beispiel 7), Tabelle 2.

Eine Verreibung von pulverförmiger Lactose und Isosorbiddinitrit in Pulverform wird in einem langsam laufenden Mischer in den angegebenen Mengenverhältnissen hergestellt und in dieser Form der Zubereitung der übrigen Stoffe nach Beispiel 8 zugegeben und eingemischt. Die Schluckkapselfüllung nach Beispiel 6 und die Depotzubereitungen für auf die Haut aufzuklebende Pflaster sind entsprechend ihrem Gehalt an Lactose nicht transparent und von salbenartig-zäher Beschaffenheit.

Tabelle 1

| Beispiele | | 1a | 1b | A | B | 2a | C | 2b | 3 | D |
|---|---|---|---|---|---|---|---|---|---|---|
| | | Mengen in Gew.-% | | | | | | | | |
| Ölbasis | Myglyol 812 * | 87,0 | 67,0 | 87,0 | 67,0 | 85,0 | 85,0 | 60,0 | 85,0 | 85,0 |
| | Myglyol 829 ** | – | 20,0 | – | 20,0 | – | – | 25,0 | – | – |
| hydrophiles, feindisp. $SiO_2$ | Aerosil 200 | 8,3 | 8,3 | 11,0 | 11,0 | 9,0 | 12,0 | 9,0 | 8,2 | 11,0 |
| hydrophobes, feindisp. $SiO_2$ | Aerosil R 992 | 2,2 | 2,7 | – | – | 3,0 | – | 3,0 | 2,8 | – |
| Nitroester | Glycerintrinitrat | 2,0 | 2,0 | 2,0 | 2,0 | – | – | – | 2,0 | 2,0 |
| | Isosorbid-dinitrat | – | – | – | – | 2,5 | 2,5 | 2,5 | 2,0 | 2,0 |
| Produkt | | transpar. Gele | | | | | transp. Gele | | | |
| Stabilität (Lagertest) | 6 Mon. 35°C Abscheidung (Gew-%) | 0,0 | 0,0 | 1,6 | 1,5 | 0,0 | 1,8 | 0,0 | 0,0 | 1,7 |
| | 12 Mon. " | 0,0 | 0,0 | 2,0 | 2,3 | 0,0 | 2,8 | 0,0 | 0,0 | 2,6 |

\* Neutralöl (Triglycerid der gesättigten Fettsäuren Capryl-/Caprinsäure

\*\* Triglycerid gesättigter, mittelkettiger Kokosölfettsäuren und der Bernsteinsäuren

Beispiele 1 bis 3 erfindungsgemäß, Vergleichsbeispiel A bis D nach Stand der Technik

|  |  | 4 | 5 | 6 | 7 | 8 |
|---|---|---|---|---|---|---|
| **Beispiele** | | | | | | |
| Ölbasis | Miglyol 812 * | - | 18,6 | 94,9 | 3,0 | 10,0 |
| | Miglyol 829 ** | 18,4 | - | - | - | - |
| | Softisan 378 **** | - | 27,9 | - | - | - |
| | Softisan 701 *** | 27,6 | - | - | - | - |
| | Softisan 601 **** | - | 46,5 | - | - | - |
| | Softisan 649 **** | 46,0 | - | 1,4 | - | - |
| | Parafinöl | - | - | - | 2,0 | 4,0 |
| hydrophiles, feindisp. $SiO_2$ | Aerosil$^{(R)}$ 200 | 4,5 | 3,8 | 1,2 | 3,0 | 4,0 |
| hydrophobes, feindisp. $SiO_2$ | Aerosil$^{(R)}$ -R 972 | 1,5 | 1,2 | 0,5 | 2,0 | 2,0 |
| fester Trägerstoff | Lactose | - | - | - | 81,0 | 56,0 |
| Nitroester | Glycerintrinitrat | 2,0 | 2,0 | - | 9,0 | - |
| | Isosorbid-dinitrat | - | - | 2,0 | - | 24,0 |
| **Produkt** | | S a l b e | | S | P | P |
| **Stabilität** | 12 Mon. 35 °C(Gew.-%) | 0,0 | 0,0 | 0,0 | 0,0 | 0,0 |

Tabelle 2

*** Triglyceridgemisch ungesättigter, hydroxylgruppenreicher Pflanzenfettsäuren

**** Triglyceridgemisch gesättigter Pflanzenfettsäuren

P = Depotzubereitung für Pflaster

S = Schluckkapselfüllung

Beispiele 4 und 6 bis 8 erfindungsgemäß

Tabelle 3

| Beispiele | Mengen in Gew.-% | | | |
|---|---|---|---|---|
| | 9 a | 9 b | 10 a | 10 b |
| Ölbasis | | | | |
| Propandiol-1,2 | 85.0 | 30.0 | 85.5 | 41.0 |
| Myglyol 812 * | – | 54.0 | – | – |
| Myglyol 829 ** | – | – | – | 45.5 |
| hydrophiles, feindisp. SiO$_2$   Aerosil 200 | 8.0 | 8.0 | 8.0 | 8.0 |
| hydrophobes, feindisp. SiO$_2$   Aerosil R 992 | 3.0 | 3.0 | 4.0 | 3.0 |
| Nitroester   Glycerintrinitrat | 4.0 | 5.0 | – | – |
| Isosorbid-dinitrat | – | – | 2.5 | 2.5 |
| Produkt | transparente Gele | | | |
| Stabilität (Lagertest)   6 Mon.   35°C Abscheidung (Gew-%) | keine Abscheidung | | | |
| 12 Mon.   " | " | | | |

\* Neutralöl (Triglycerid der gesättigten Fettsäuren Capryl-/Caprinsäure

\*\* Triglycerid gesättigter, mittelkettiger Kokosölfettsäuren und der Bernsteinsäuren

Beispiele 1 bis 10 erfindungsgemäß, Vergleichsbeispiel A bis D nach Stand der Technik

## Patentansprüche

1. Ohne Zusatz von Wasser hergestellte Gel-Zubereitung oder gelhaltige Zubereitung, enthaltend koronarwirksame Nitroester, eine Ölbasis und feindisperses SiO$_2$ sowie ggf. Hilfsstoffe im Verhältnis von

   a) 0,5 bis 25,0 Gew.-Teilen eines Nitroesters zu

   b) 55,0 bis 90,0 Gew.-Teilen einer Ölbasis zu

   c) 5,0 bis 40,0 Gew.-Teilen feindisperses SiO$_2$,

   **gekennzeichnet dadurch,** daß das feindisperse SiO$_2$ aus

$c_1$: 95,0 bis 60,0 Gew.-% hydrophilem $SiO_2$ und

$c_2$: 5,0 bis 40,0 Gew.-% hydrophobem $SiO_2$

besteht.

2. Zubereitung nach Anspruch 1, dadurch gekennzeichnet, daß das feindisperse $SiO_2$ aus

$c_1$: 80 bis 60 Gew.-% hydrophilem $SiO_2$ und

$c_2$: 20 bis 40 Gew.-% hydrophobem $SiO_2$

besteht.

3. Zubereitung nach einem der Ansprüche 1 oder 2, dadurch gekennzeichnet, daß sie 0,5 bis 25,0 Gew.-% leichtlösliche Nitroester gelöst enthält.

4. Zubereitung nach einem der Ansprüche 1 oder 2, dadurch gekennzeichnet, daß sie 0,5 bis 10,0 Gew.-% schwerlösliche Nitroester gelöst enthält.

5. Zubereitung nach einem der Ansprüche 1 bis 4, dadurch gekennzeichnet, daß Nitroester als Suspension oder absorbiert an feste Trägerstoffe und ggf. zusätzlich gelöste Nitroester enthalten sind.

6. Zubereitung nach einem der Ansprüche 1 bis 5, gekennzeichnet durch zusätzliche Anwesenheit eines festen Trägerstoffes für die Nitroester in Mengen von bis zu 85 Gew.-%, bezogen auf die Summe der Bestandteile a) bis c) der Zubereitung.

7. Zubereitung nach mindestens einem der Ansprüche 1 bis 6, dadurch gekennzeichnet, daß die Ölbasis aus einer für pharmazeutische Zwecke verwendbaren öligflüssigen Substanz, die zwischen - 10 °C und + 30 °C flüssig ist, besteht.

8. Zubereitung nach Anspruch 7, dadurch gekennzeichnet, daß die Ölbasis aus einem Triglycerid der gesättigten geradkettigen Fettsäuren der Kettenlänge $C_8$ bis $C_{12}$ besteht.

9. Verfahren zur Herstellung der Zubereitung nach einem der Ansprüche 1 bis 8, dadurch gekennzeichnet, daß die Lösung mindestens eines Nitroesters in der Ölbasis oder die Suspension des Nitroesters oder die Suspension des an einen Trägerstoff absorbierten Nitroesters gleichzeitig oder nacheinander mit dem hydrophilen $SiO_2$ und dem hydrophoben $SiO_2$ sowie weiteren Bestandteilen unter Rühren gleichmäßig, ggf. unter vermindertem Druck, vermischt werden, darauf unter vermindertem Druck gerührt und entlüftet wird, bis eine Gel-Zubereitung von gleichbleibender Viskosität erreicht ist.

10. Ohne Zusatz von Wasser hergestellte Gelzubereitung oder gelhaltige Zubereitung, enthaltend coronar wirksame Nitroester, eine Ölbasis und feindisperses $SiO_2$ sowie gegebenenfalls Hilfsstoffe, dadurch gekennzeichnet, daß sie

3 Gew.-%　　eines Triglycerids der gesättigten Fettsäuren Capryl-/Caprinsäure und

2 Gew.-%　　Paraffinöl als Ölbasis,

3 Gew.-%　　hydrophiles $SiO_2$ und

2 Gew.-%　　hydrophobes $SiO_2$

als feindisperses $SiO_2$,

9 Gew.-%　　Glycerintrinitrat als Nitroester sowie

81 Gew.-%　　Lactose als Trägerstoff

enthält.

11. Ohne Zusatz von Wasser hergestellte Gelzubereitung oder gelhaltige Zubereitung, enthaltend coronar wirksame Nitroester, eine Ölbasis und feindisperses $SiO_2$ sowie gegebenenfalls Hilfsstoffe, dadurch gekennzeichnet, daß sie

94,9 Gew.-%　　eines Triglycerids der gesättigten Fettsäuren Capryl-/Caprinsäure und

1,4 Gew.-%　　eines Triglyceridgemisches gesättigter Pflanzenfettsäuren als Ölbasis,

1,2 Gew.-%　　hydrophiles $SiO_2$ und

0,5 Gew.-%　　hydrophobes $SiO_2$

als feindisperses $SiO_2$ sowie

2 Gew.-%　　Isosorbit-Dinitrat als Nitroester

enthält.

9

**Claims**

1. Gel preparation or gel containing preparation produced without addition of water, containing coronary effective nitroesters, an oil base and finely dispersed $SiO_2$, as well as, optionally, auxiliary substances, in the ratio of
   a) 0.5 to 25.0 parts by weight of a nitroester to
   b) 55.0 to 90.0 parts by weight of an oil base to
   c) 5.0 to 40.0 parts by weight of finely dispersed $SiO_2$,
   characterised in that the finely dispersed $SiO_2$ consists of
   $c_1$: 95.0 to 60.0 % by weight of hydrophilic $SiO_2$ and
   $c_2$: 5.0 to 40.0 % by weight of hydrophobic $SiO_2$.

2. Preparation according to claim 1, characterised in that the finely dispersed $SiO_2$ consists of $c_1$: 80 to 60 % by weight of hydrophilic $SiO_2$ and $c_2$: 20 to 40 % by weight of hydrophobic $SiO_2$.

3. Preparation according to one of claims 1 or 2, characterised in that it contains dissolved 0.5 to 25.0 % of readily soluble nitroesters.

4. Preparation according to one of claims 1 or 2, characterised in that it contains dissolved 0.5 to 10.0 % by weight of hardly soluble nitroesters.

5. Preparation according to one of claims 1 to 4, characterised in that nitroesters are contained as suspension or absorbed on solid carrier substances and optionally, additionally, dissolved nitroesters are contained.

6. Preparation according to one of claims 1 to 5, characterised by additional presence of a solid carrier substance for the nitroesters in amounts of up to 85% by weight related to the sum of constituents a) to c) of the preparation.

7. Preparation according to at least one of claims 1 to 6, characterised in that the oil base consists of an oleaginous substance usable for pharmaceutical purposes which is liquid between -10°C and +30°C.

8. Preparation according to claim 7, characterised in that the oil base consists of a triglyceride of saturated straight-chain fatty acids of chain length $C_8$ to $C_{12}$.

9. Process for the manufacture of the preparation according to one of claims 1 to 8, characterised in that the solution at least of one nitroester in the oil base or the suspension of the nitroester or the suspension of the nitroester absorbed on a carrier substance are mixed simultaneously or successively with the hydrophilic $SiO_2$ and the hydrophobic $SiO_2$, as well as further constituents, and stirred uniformly, optionally under reduced pressure, then there is stirring under reduced pressure and deaeration until a gel preparation of constant viscosity is attained.

10. Gel preparation or gel containing preparation produced without addition of water, containing coronary effective nitroesters, an oil base and finely dispersed $SiO_2$, as well as, optionally, auxiliary substances, characterised in that it contains
    3% by weight    of a triglyceride of the saturated fatty acids caprylic/caprinic acid and
    2% by weight    paraffin oil as oil base,
    3% by weight    of hydrophilic $SiO_2$ and
    2% by weight    of hydrophobic $SiO_2$ as finely dispersed $SiO_2$,
    9% by weight    glycerine trinitrate as nitroester as well as
    81% by weight   of lactose as carrier substance.

11. Gel preparation or gel containing preparation produced without addition of water, containing coronary effective nitroesters, an oil base and finely dispersed $SiO_2$, as well as, optionally, auxiliary substances, charcterised in that it contains
    94.9% by weight    of a triglyceride of the saturated fatty acids caprylic/caprinic acid and
    1.4% by weight     of a triglyceride mixture of saturated vegetable fatty acids as oil base,
    1.2% by weight     of hydrophilic $SiO_2$ and

10

0.5% by weight      of hydrophobic SiO$_2$ as finely dispersed SiO$_2$ as well as

2 % by weight of    isosorbitol-dinitrate as nitroester.

## Revendications

1.  Préparation sous forme de gel ou contenant un gel, préparée sans addition d'eau, contenant un ou des nitroester(s) actif(s) sur les coronaires, une base huileuse et de la SiO$_2$ finement dispersée, ainsi que d'éventuels adjuvants, en les proportions suivantes :

    a) 0,5 à 25,0 parties en poids de nitroesters pour

    b) 55,0 à 90,0 parties en poids d'une base huileuse pour

    c) 5,0 à 40,0 parties en poids de SiO$_2$ finement dispersée,

    **caractérisée** en ce que la SiO$_2$ finement dispersée est constituée de

    $c_1$ :     95,0 à 60,0% en poids de SiO$_2$ hydrophile et

    $c_2$ :     5,0 à 40,0% en poids de SiO$_2$ hydrophobe.

2.  Préparation conforme à la revendication 1, **caractérisée** en ce que la SiO$_2$ finement dispersée est constituée de

    $c_1$ :     80 à 60% en poids de SiO$_2$ hydrophile et

    $c_2$ :     20 à 40% en poids de SiO$_2$ hydrophobe.

3.  Préparation conforme à l'une des revendications 1 et 2, **caractérisée** en ce qu'elle contient, à l'état dissous, de 0,5 à 25,0% en poids de nitroesters facilement solubles.

4.  Préparation conforme à l'une des revendications 1 et 2, **caractérisée** en ce qu'elle contient, à l'état dissous, de 0,5 à 10,0% en poids de nitroesters diffilement solubles.

5.  Préparation conforme à l'une des revendications 1 à 4, **caractérisée** en ce qu'elle contient, des nitroesters sous forme de suspension ou sous forme absorbée sur un support solide, et le cas échéant, des nitroesters supplémentaires dissous.

6.  Préparation conforme à l'une des revendications 1 à 5, **caractérisée** par la présence supplémentaire d'un support solide pour les nitroesters, en une quantité valant jusqu'à 85% en poids, par rapport à la quantité totale des constituants a) à c) de la préparation.

7.  Préparation conforme à l'une au moins des revendications 1 à 6, **caractérisée** en ce que la base huileuse est constituée d'une substance liquide huileuse utilisable à des fins pharmaceutiques, qui est liquide entre -10°C et +30°C.

8.  Préparation conforme à la revendication 7, **caractérisée** en ce que la base huileuse est constituée d'un triglycéride d'acides gras à chaîne droite saturée, comportant de 8 à 12 atomes de carbone.

9.  Procédé de fabrication d'une préparation conforme à l'une des revendications 1 à 8, **caractérisé** en ce que l'on mélange jusqu'à homogénéité, sous agitation et éventuellement sous pression réduite, une solution d'au moins un nitroester dans la base huileuse ou une suspension de nitroester ou une suspension de nitroester absorbé sur un support, simultanément ou successivement, avec la SiO$_2$ hydrophile et la SiO$_2$ hydrophobe, ainsi qu'avec les autres constituants, puis on agite et désaère le mélange sous pression réduite, jusqu'à ce que l'on obtienne une préparation sous forme de gel de viscosité constante.

10. Préparation sous forme de gel ou contenant un gel, préparée sans addition d'eau, contenant un ou des nitroester(s) actif(s) sur les coronaires, une base huileuse et de la SiO$_2$ finement dispersée, ainsi que d'éventuels adjuvants, **caractérisée** en ce qu'elle contient

    3%      en poids d'un triglycéride d'acide gras saturé caprylique/caprique et

    2%      en poids d'huile de paraffine, comme base huileuse,

    3%      en poids de SiO$_2$ hydrophile et

    2%      en poids de SiO$_2$ hydrophobe, en tant que SiO$_2$ finement dispersée,

    9%      en poids de trinitrate de glycérine, en tant que nitroester, ainsi que

    81%     en poids de lactose, en tant que support.

**11.** Préparation sous forme de gel ou contenant un gel, préparée sans addition d'eau, contenant un ou des nitroester(s) actif(s) sur les coronaires, une base huileuse et de la $SiO_2$ finement dispersée, ainsi que d'éventuels adjuvants, **caractérisée** en ce qu'elle contient

| | |
|---|---|
| 94,9% | en poids d'un triglycéride d'acide gras saturé caprylique/caprique et |
| 1,4% | en poids d'un mélange de triglycérides d'acides gras végétaux saturés, |
| 1,2% | en poids de $SiO_2$ hydrophile et |
| 0,5% | en poids de $SiO_2$ hydrophobe, en tant que $SiO_2$ finement dispersée, |
| 2% | en poids de dinitrate d'isosorbitol, en tant que nitroester. |